# EUROPEAN PATENT APPLICATION

(11) **EP 4 821 590 A1**
(43) Date of publication of application: **14.10.2026**
(21) Application number: 24900282.5
(22) Date of filing: 08.10.2024
(51) Int. Cl.: B01F 35/221, B01F 21/00, B01F 35/82, B01F 35/213, C12M 1/00, C12N 1/00

(54) **SOLUTION PREPARATION DEVICE, METHOD FOR OPERATING SOLUTION PREPARATION DEVICE, AND PROGRAM FOR OPERATING SOLUTION PREPARATION DEVICE**

(30) Priority: 05.12.2023 JP 2023205644
(71) Applicant: FUJIFILM Corporation, Tokyo 106-8620 (JP)
(72) Inventor: YAMAMOTO, Takashi, Tokyo 106-8620 (JP); HASEGAWA, Masataka, Tokyo 106-8620 (JP); INABA, Tatsuya, Tokyo 106-8620 (JP)
(74) Representative: Klunker IP Patentanwälte PartG mbB
(86) International application number: PCT/JP2024/036007
(87) International publication number: WO 2025/120985

(57) **Abstract**

A solution preparation device that continuously supplies a solvent and a powder to a mixing container to continuously prepare a solution having a set concentration in the mixing container and that continuously supplies the solution having the set concentration from the mixing container to a processing container, the solution preparation device including: a processor, in which the processor is configured to: perform concentration adjustment processing for maintaining the set concentration on a solution remaining in the mixing container in a case where the continuous supply of the powder to the mixing container is stopped; and supply the solution having the set concentration, on which the concentration adjustment processing is performed, to the processing container to satisfy a required amount of the solution having the set concentration.

## Description

### Technical Field

The technology of the present disclosure relates to a solution preparation device, an operation method of a solution preparation device, and an operation program of a solution preparation device.

### Related Art

For example, in a bioprocess such as culturing Chinese hamster ovary cells into which an antibody gene has been incorporated to obtain an antibody drug, various solutions are required. WO2021/123248A discloses a solution preparation device that continuously supplies a solvent and a powder to a mixing container and continuously prepares a solution having a set concentration by stirring and mixing the solvent and the powder in the mixing container. The mixing container is connected to a processing container, and the solution is continuously supplied from the mixing container to the processing container. The solvent is, for example, purified water such as pure water, the powder is, for example, a powdered culture medium, and the solution is, for example, a culture medium (culture solution). In addition, the processing container is, for example, a culture tank.

### SUMMARY OF THE INVENTION

### Technical Problem

In the solution preparation device, in a case where the processing in the processing container is ended and the required amount of the solution is completely supplied from the mixing container to the processing container, the continuous supply of the solvent and the powder to the mixing container is stopped. The preparation of the solution having the set concentration in the mixing container may be continued until the required amount of the solution is completely supplied from the mixing container to the processing container. In this case, at a point in time at which the continuous supply of the solvent and the powder to the mixing container is stopped, the solution remains in the mixing container. Then, the solution remaining in the mixing container is completely discarded without being supplied to the processing container. Therefore, many resources, particularly the powder, are wasted.

A measure of reducing the wasted powder by supplying a part of the solution remaining in the mixing container after the supply of the solvent and the powder is stopped to the processing container to add the required amount is considered. However, the solution remaining in the mixing container after the supply of the solvent and the powder is stopped has a higher concentration than the set concentration with the passage of time because the undissolved powder is gradually dissolved. Therefore, the solution remaining in the mixing container after the supply of the solvent and the powder is stopped cannot be supplied to the processing container as it is.

One embodiment according to the technology of the present disclosure provides a solution preparation device, an operation method of a solution preparation device, and an operation program of a solution preparation device capable of reducing a cost by reducing wasted powder.

### Solution to Problem

A solution preparation device according to the present disclosure is a solution preparation device that continuously supplies a solvent and a powder to a mixing container to continuously prepare a solution having a set concentration in the mixing container and that continuously supplies the solution having the set concentration from the mixing container to a processing container, the solution preparation device comprising: a processor, in which the processor is configured to: perform concentration adjustment processing for maintaining the set concentration on a solution remaining in the mixing container in a case where the continuous supply of the powder to the mixing container is stopped; and supply the solution having the set concentration, on which the concentration adjustment processing is performed, to the processing container to satisfy a required amount of the solution having the set concentration.

It is preferable that the processor is configured to perform, as the concentration adjustment processing, processing of diluting the solution by supplying the solvent to the mixing container.

It is preferable that the processor is configured to perform, as the concentration adjustment processing, processing of gradually decreasing a supply amount of the powder.

It is preferable that the processor is configured to perform the concentration adjustment processing according to a measurement result of a concentration of the solution.

It is preferable that the measurement result is based on spectroscopic spectral data of the solution.

It is preferable that a measuring instrument for the spectroscopic spectral data is installed in the mixing container.

It is preferable that the solvent is purified water, the powder is a powdered culture medium, and the solution is a culture medium.

An operation method of a solution preparation device according to the present disclosure is an operation method of a solution preparation device that continuously supplies a solvent and a powder to a mixing container to continuously prepare a solution having a set concentration in the mixing container and that continuously supplies the solution having the set concentration from the mixing container to a processing container, the method comprising: performing concentration adjustment processing for maintaining the set concentration on a solution remaining in the mixing container in a case where the continuous supply of the powder to the mixing container is stopped; and supplying the solution having the set concentration, on which the concentration adjustment processing is performed, to the processing container to satisfy a required amount of the solution having the set concentration.

An operation program of a solution preparation device according to the present disclosure is an operation program of a solution preparation device that continuously supplies a solvent and a powder to a mixing container to continuously prepare a solution having a set concentration in the mixing container and that continuously supplies the solution having the set concentration from the mixing container to a processing container, the program causing a computer to execute a process comprising: performing concentration adjustment processing for maintaining the set concentration on a solution remaining in the mixing container in a case where the continuous supply of the powder to the mixing container is stopped; and supplying the solution having the set concentration, on which the concentration adjustment processing is performed, to the processing container to satisfy a required amount of the solution having the set concentration.

### Advantageous Effects of Invention

According to the technology of the present disclosure, it is possible to provide a solution preparation device, an operation method of a solution preparation device, and an operation program of a solution preparation device capable of reducing a cost by reducing wasted powder.

### BRIEF DESCRIPTION OF THE DRAWINGS

FIG. 1 is a diagram showing a culture medium preparation device and a culture tank.
FIG. 2 is a block diagram showing a configuration of a control unit.
FIG. 3 is a block diagram showing a processing unit of a CPU.
FIG. 4 is a diagram showing processing of a concentration prediction unit.
FIG. 5 is an explanatory diagram of processing in the related art.
FIG. 6 is an explanatory diagram of processing of a first embodiment.
FIG. 7 is an explanatory diagram of processing of a second embodiment.

### DESCRIPTION OF EMBODIMENTS

### [First embodiment]

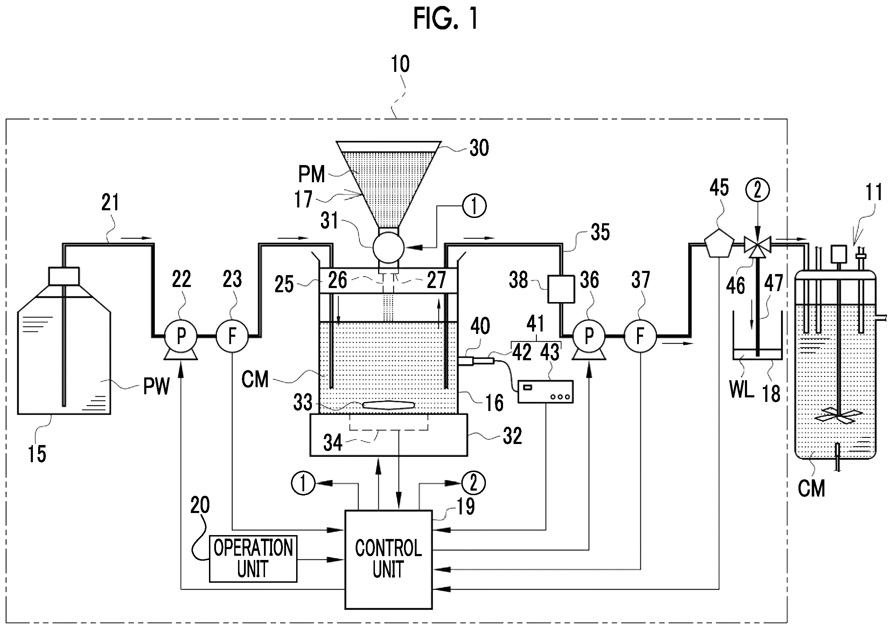

As shown in FIG. 1 as an example, a culture medium preparation device 10 prepares a culture medium CM. The culture medium preparation device 10 is connected to the culture tank 11, and continuously supplies the prepared culture medium CM to the culture tank 11. The culture medium preparation device 10 is an example of a "solution preparation device" according to the technology of the present disclosure. The culture tank 11 is an example of a "processing container" according to the technology of the present disclosure. In addition, the culture medium CM is an example of a "solution" according to the technology of the present disclosure. It is noted that in FIG. 1, an example in which the culture medium preparation device 10 is directly connected to the culture tank 11 has been described, but the present disclosure is not limited thereto. A tank for temporarily storing the culture medium CM may be provided between the culture medium preparation device 10 and the culture tank 11.

For example, Chinese hamster ovary cells into which an antibody gene has been incorporated are seeded in the culture tank 11. In the culture tank 11, the Chinese hamster ovary cells are cultured in the culture medium CM for a determined culture period, for example, one month. As described above, since the culture medium CM is continuously supplied to the culture tank 11 from the culture medium preparation device 10, the culture of the Chinese hamster ovary cells performed in the culture tank 11 is a perfusion culture. The culture tank 11 is connected to a purification device (not shown). The purification device purifies an antibody produced by Chinese hamster ovary cells in the culture process, and yields the antibody as an active pharmaceutical ingredient of an antibody drug.

The culture medium preparation device 10 comprises a pure water storage tank 15, a mixing container 16, a powdered culture medium supply machine 17, a waste liquid recovery tank 18, a control unit 19, an operation unit 20, and the like.

The pure water storage tank 15 stores pure water PW. The pure water PW is an example of a "solvent" and "purified water" according to the technology of the present disclosure. One end of a pure water supply line 21 is connected to the pure water storage tank 15. The other end of the pure water supply line 21 is connected to the mixing container 16. A pure water supply pump 22 and a pure water flow meter 23 are provided in the pure water supply line 21. The pure water supply pump 22 is driven and controlled by the control unit 19. In a case where the pure water supply pump 22 is driven, the pure water PW in the pure water storage tank 15 is supplied to the mixing container 16 through the pure water supply line 21. The pure water flow meter 23 is disposed downstream of the pure water supply pump 22, and measures an inflow amount of the pure water PW that passes through the pure water supply line 21 and flows into the mixing container 16. The pure water flow meter 23 outputs the measured inflow amount to the control unit 19. The control unit 19 controls the driving of the pure water supply pump 22 such that the inflow amount of the pure water flow meter 23 is a set inflow amount.

The mixing container 16 is a container for mixing the pure water PW and the powdered culture medium PM to generate the culture medium CM having a set concentration, and has, for example, a wide cylindrical shape. The mixing container 16 has a capacity of, for example, about 0.001 times to 0.1 times the amount of the culture medium CM required in the culture tank 11 for the entire culture period, that is, the required amount of the culture medium CM. With the mixing container 16 having a capacity smaller than the required amount of the culture medium CM, it is possible to reduce the installation area. However, since the capacity of the mixing container 16 is smaller than the required amount of the culture medium CM, the preparation of the culture medium CM in the mixing container 16 is continued until the required amount of the culture medium CM is completely supplied from the mixing container 16 to the culture tank 11.

The upper portion of the mixing container 16 is sealed with a lid 25. A through-hole 26 is formed in a center portion of the lid 25. A discharge port 27 for the powdered culture medium PM of the powdered culture medium supply machine 17 is aseptically connected to an upper end part of the through-hole 26. As a result, the mixing container 16 and the powdered culture medium supply machine 17 are aseptically connected. The powdered culture medium PM discharged from the discharge port 27 is supplied to the mixing container 16 through the through-hole 26. The powdered culture medium PM is an example of a "powder" according to the technology of the present disclosure.

The powdered culture medium supply machine 17 is composed of a funnel-shaped hopper 30 that stores the powdered culture medium PM and a feeder 31 that continuously discharges the powdered culture medium PM in the hopper 30 from the discharge port 27. The hopper 30 has a funnel shape, and a sterile bag (not shown) in which the powdered culture medium PM is accommodated is set in an upper opening of the hopper 30. The powdered culture medium PM is introduced into the hopper 30 from the sterile bag without coming into contact with outside air. As a result, the sterile state of the powdered culture medium PM is maintained.

The feeder 31 is attached to a lower portion of the hopper 30 in which a diameter is narrowed. The feeder 31 is driven and controlled by the control unit 19, and continuously discharges the set amount of the powdered culture medium PM from the discharge port 27. The feeder 31 is, for example, a screw feeder (also referred to as an auger feeder) that continuously discharges the powdered culture medium PM in a set amount from the discharge port 27 by rotationally driving a spiral screw with a motor under the control of the control unit 19. Although not shown, a stirrer for crushing rat holes generated by long-term use is introduced into the hopper 30. The inner wall of the hopper 30 may be subjected to a sliding treatment for improving the discharge properties of the powdered culture medium PM. In addition, the feeder 31 is not limited to the screw feeder, and may be a table feeder (also referred to as a circle feeder), a rotary feeder, a belt feeder, a vibrating feeder, or the like.

The mixing container 16 is installed on the stirrer 32. A stirring bar 33 is put into the mixing container 16. The stirring bar 33 has an elongated cocoon-like shape in which both end portions are rounded and a central portion is slightly swollen. A magnet is built in the stirring bar 33. The stirrer 32 is driven and controlled by the control unit 19. The stirrer 32 generates a magnetic force for rotating the stirring bar 33. The stirrer 32 rotates the stirring bar 33 at a set stirring rotation speed to mix the pure water PW and the powdered culture medium PM to obtain a culture medium CM. It should be noted that, without being limited to the stirring bar 33, the pure water PW and the powdered culture medium PM may be mixed by rotating a stirring blade (also referred to as a stirring vane).

A weighing scale 34 is built in the stirrer 32. The weighing scale 34 measures the weight of the mixing container 16 installed on the stirrer 32. The weighing scale 34 outputs the measured weight to the control unit 19. The control unit 19 controls the driving of the powdered culture medium supply machine 17 such that the weight is a set amount. It should be noted that the supply amount of the powdered culture medium PM related to the weight of the mixing container 16 is set based on the set concentration of the culture medium CM to be prepared, the outflow amount of the culture medium CM to the culture tank 11, and the like.

One end of a culture medium supply line 35 is connected to the mixing container 16. The other end of the culture medium supply line 35 is connected to the culture tank 11. A culture medium supply pump 36 and a culture medium flow meter 37 are provided in the culture medium supply line 35. The culture medium supply pump 36 is driven and controlled by the control unit 19. In a case where the culture medium supply pump 36 is driven, the culture medium CM in the mixing container 16 is supplied to the culture tank 11 through the culture medium supply line 35. The culture medium flow meter 37 is disposed downstream of the culture medium supply pump 36, and measures the outflow amount of the culture medium CM that passes through the culture medium supply line 35 and flows out to the culture tank 11. The culture medium flow meter 37 outputs the measured outflow amount to the control unit 19. The control unit 19 controls the driving of the culture medium supply pump 36 such that the outflow amount of the culture medium flow meter 37 is the set outflow amount.

The inflow amount of the pure water PW into the mixing container 16 and the outflow amount of the culture medium CM from the mixing container 16 are set to values such that the amount of the liquid (the culture medium CM including the undissolved powdered culture medium PM) in the mixing container 16 does not change from the beginning to the end. More specifically, the outflow amount of the culture medium CM is set according to the amount obtained by combining the inflow amount of the pure water PW and the supply amount of the powdered culture medium PM. In practice, the weight of the mixing container 16 is measured by the weighing scale 34, and the inflow amount of the pure water PW and the outflow amount of the culture medium CM are set such that the measured weight is the set amount.

Here, the required amount of the culture medium CM can be easily calculated by multiplying the amount of the culture medium CM required for culturing in a unit time by the culture period. The control unit 19 integrates the outflow amount of the culture medium CM measured by the culture medium flow meter 37. Then, in a case where the integrated value of the outflow amount is the required amount, the supply of the culture medium CM from the mixing container 16 to the culture tank 11 is stopped.

A filter 38 is disposed upstream of the culture medium supply pump 36 of the culture medium supply line 35. The filter 38 removes unnecessary substances in the culture medium CM. The unnecessary substance is, for example, an undissolved powdered culture medium PM, and the like. In a case where the amount of the unnecessary substances such as the undissolved powdered culture medium PM can be set to be less than the allowable amount, the filter 38 may not be provided.

A connector part 40 is provided in the mixing container 16. A sensor unit 42 of a Raman spectrometer 41 is connected to the connector part 40. The Raman spectrometer 41 is a device that evaluates a substance by using characteristics of Raman scattered light, and is an example of a "measuring instrument" according to the technology of the present disclosure. In a case where the substance is irradiated with excitation light, the Raman scattered light having a different wavelength from the excitation light is generated by an interaction between the excitation light and the substance. A wavelength difference between the excitation light and the Raman scattered light corresponds to an energy of molecular vibration of the substance. Therefore, the Raman scattered light having different wave numbers can be obtained between the substances having different molecular structures. Out of a Stokes line and an anti-Stokes line, it is preferable to use the Stokes line as the Raman scattered light.

The Raman spectrometer 41 is composed of the sensor unit 42 and an analyzer 43. The sensor unit 42 is connected to the connector part 40, and a distal end thereof is immersed in the culture medium CM in the mixing container 16. The sensor unit 42 emits the excitation light from the distal end. The culture medium CM is irradiated with the excitation light. The Raman scattered light is generated by an interaction between the excitation light and a component such as the powdered culture medium PM dissolved in the culture medium CM. The sensor unit 42 receives the Raman scattered light and guides the received Raman scattered light to the analyzer 43.

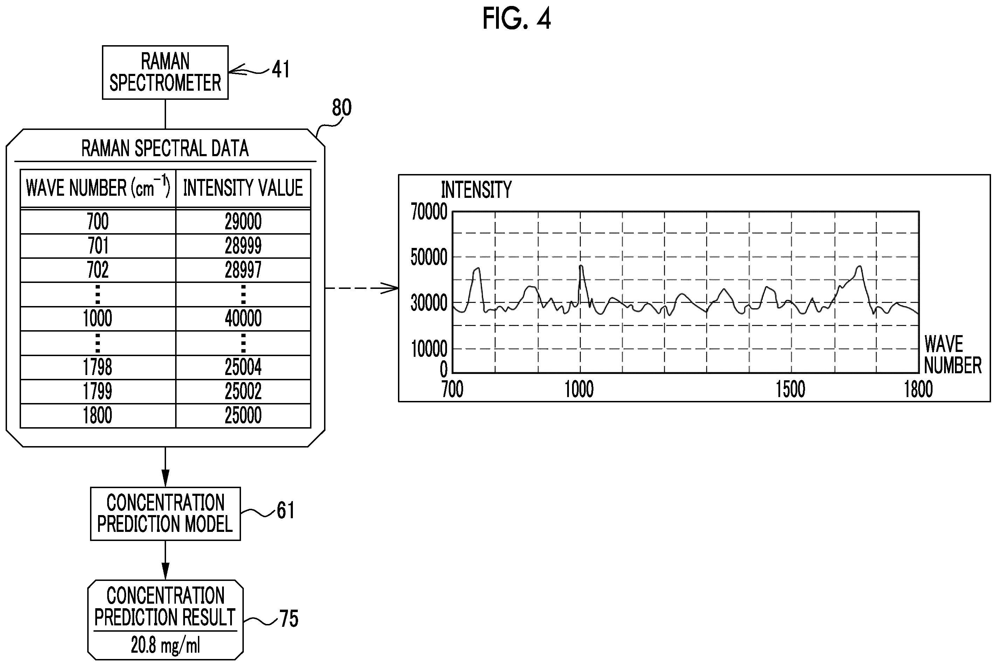

The analyzer 43 decomposes the Raman scattered light for each wave number and derives an intensity value of the Raman scattered light for each wave number to generate Raman spectral data 80 (see FIG. 4) representing a spectrum of the Raman scattered light, that is, a Raman spectrum. The Raman spectral data 80 is data in which the intensity value of the Raman scattered light RSL is registered for each wave number. The Raman spectral data 80 is used to predict a concentration of the culture medium CM as described later. The Raman spectral data 80 is an example of "spectroscopic spectral data" according to the technology of the present disclosure.

A hydrogen-ion exponent meter 45 is disposed downstream of the culture medium flow meter 37 of the culture medium supply line 35. In addition, a waste liquid line 47 is connected to the downstream of the hydrogen-ion exponent meter 45 of the culture medium supply line 35 via a three-way valve 46. The three-way valve 46 is driven and controlled by the control unit 19. The waste liquid recovery tank 18 is connected to the waste liquid line 47. The waste liquid recovery tank 18 collects the waste liquid WL such as the culture medium CM that is not at the set concentration or the culture medium CM having a hydrogen-ion exponent of an abnormal value.

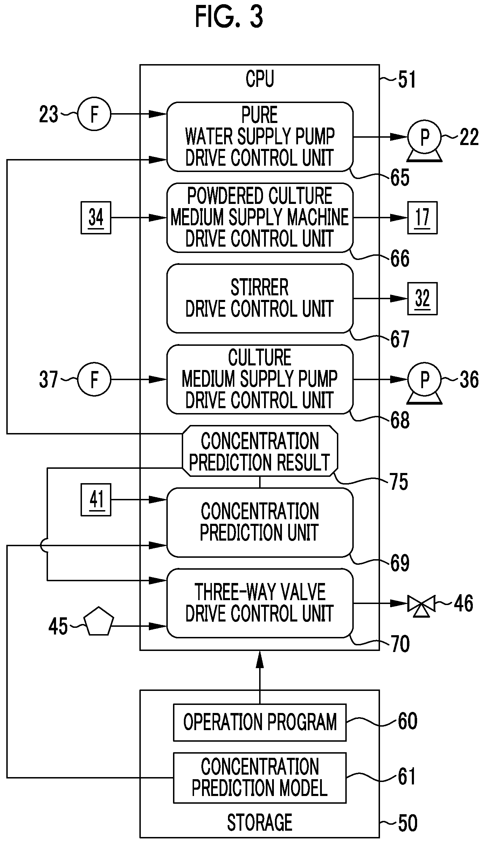

The control unit 19 determines whether or not the culture medium CM is at the set concentration from a concentration prediction result 75 (see FIG. 3) of the culture medium CM based on the Raman spectral data 80. In a case where it is determined that the culture medium CM is not at the set concentration, the control unit 19 sets the flow path of the three-way valve 46 to the waste liquid line 47 side, and discharges the waste liquid WL, which is the culture medium CM not having the set concentration, to the waste liquid recovery tank 18. On the other hand, in a case where it is determined that the culture medium CM has the set concentration, the control unit 19 sets the flow path of the three-way valve 46 to the culture tank 11 side, and introduces the culture medium CM having the set concentration into the culture tank 11. In this way, only the culture medium CM having the set concentration is supplied to the culture tank 11. It is possible to prevent a problem such as the supply of the culture medium CM not having the set concentration to the culture tank 11 and the failure of the culture of the Chinese hamster ovary cells.

The hydrogen-ion exponent meter 45 measures the hydrogen-ion exponent of the culture medium CM that passes through the culture medium supply line 35. The hydrogen-ion exponent meter 45 outputs the measured hydrogen-ion exponent to the control unit 19. In a case where the hydrogen-ion exponent from the hydrogen-ion exponent meter 45 is an abnormal value, the control unit 19 sets the flow path of the three-way valve 46 to the waste liquid line 47 side, and discharges the waste liquid WL, which is the culture medium CM having an abnormal value of the hydrogen-ion exponent, to the waste liquid recovery tank 18.

The operation unit 20 is, for example, a touch panel, and receives various operation instructions from an operator of the culture medium preparation device 10. The various operation instructions include a preparation start instruction for the culture medium CM, a forced stop instruction for the supply of the culture medium CM from the mixing container 16 to the culture tank 11, and the like.

The pure water storage tank 15, the mixing container 16, the hopper 30 and the feeder 31 of the powdered culture medium supply machine 17, and the waste liquid recovery tank 18 are disposable in one time, that is, so-called single-use. In addition, the pure water supply line 21, the culture medium supply line 35, the filter 38, the three-way valve 46, and the waste liquid line 47 are also single-use.

All flow paths from the pure water storage tank 15 to the culture tank 11 through the pure water supply line 21, the mixing container 16, and the culture medium supply line 35 are aseptically connected. In addition, the culture medium supply line 35, the three-way valve 46, and the waste liquid line 47 are also aseptically connected.

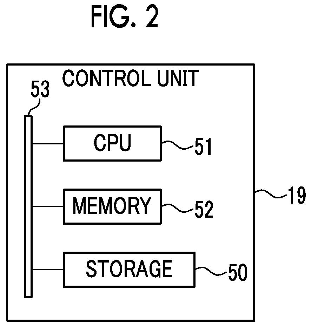

As shown in FIG. 2 as an example, the control unit 19 comprises a storage 50, a central processing unit (CPU) 51, and a memory 52. The storage 50, the CPU 51, and the memory 52 are connected to each other through a busline 53. The control unit 19 is an example of a "computer" according to the technology of the present disclosure.

The storage 50 is a hard disk drive or a solid-state drive. The storage 50 stores, for example, a control program, such as an operating system, various application programs, and various types of data associated with these programs.

The memory 52 is a work memory for executing processing via the CPU 51. The CPU 51 loads a program stored in the storage 50 into the memory 52 and executes processing corresponding to the program. Therefore, the CPU 51 controls each unit of the computer comprehensively. The CPU 51 is an example of a "processor" according to the technology of the present disclosure. The memory 52 may be incorporated in the CPU 51.

As shown in FIG. 3 as an example, the storage 50 stores an operation program 60. The operation program 60 is an example of an "operation program of a solution preparation device" according to the technology of the present disclosure. In addition to the operation program 60, the concentration prediction model 61 is also stored in the storage 50.

In a case where the operation program 60 is activated, the CPU 51 functions as a pure water supply pump drive control unit 65, a powdered culture medium supply machine drive control unit 66, a stirrer drive control unit 67, a culture medium supply pump drive control unit 68, a concentration prediction unit 69, and a three-way valve drive control unit 70.

The pure water supply pump drive control unit 65 controls the drive of the pure water supply pump 22 based on the inflow amount of the pure water PW into the mixing container 16 measured by the pure water flow meter 23. In addition, the pure water supply pump drive control unit 65 controls the drive of the pure water supply pump 22 based on a concentration prediction result 75 by the concentration prediction unit 69. The drive control of the pure water supply pump 22 based on the concentration prediction result 75 will be described later.

The powdered culture medium supply machine drive control unit 66 controls the drive of the powdered culture medium supply machine 17 based on the weight of the mixing container 16 measured by the weighing scale 34. The stirrer drive control unit 67 controls the drive of the stirrer 32 such that the stirring rotation speed of the stirring bar 33 is the set rotation speed.

The culture medium supply pump drive control unit 68 controls the drive of the culture medium supply pump 36 based on the outflow amount of the culture medium CM from the mixing container 16 measured by the culture medium flow meter 37. The concentration prediction unit 69 predicts the concentration of the culture medium CM from the Raman spectral data 80 using the concentration prediction model 61, and outputs the concentration prediction result 75, which is the result, to the pure water supply pump drive control unit 65 and the three-way valve drive control unit 70. The concentration prediction result 75 is an example of a "measurement result" according to the technology of the present disclosure.

The three-way valve drive control unit 70 controls the drive of the three-way valve 46 based on the concentration prediction result 75 by the concentration prediction unit 69 and the hydrogen-ion exponent of the culture medium CM measured by the hydrogen-ion exponent meter 45.

As shown in FIG. 4 as an example, the concentration prediction unit 69 inputs the Raman spectral data 80 from the Raman spectrometer 41 to the concentration prediction model 61. Then, the concentration prediction result 75 is output from the concentration prediction model 61. The concentration prediction model 61 is, for example, a machine learning model such as a neural network or a support vector machine, and is trained in advance to output the concentration prediction result 75 in response to the input of the Raman spectral data 80.

In the present example, the Raman spectral data 80 is data in which intensity values of the Raman scattered light RSL in a range of wave numbers of 700 cm⁻¹ to 1800 cm⁻¹ are derived in increments of 1 cm⁻¹. In addition, in the present example, the unit of the concentration prediction result 75 is mg/ml. It should be noted that a graph shown on the right side of the Raman spectral data 80 is a graph in which the intensity value of the Raman spectral data 80 is plotted for each wave number and the plotted points are connected with a line.

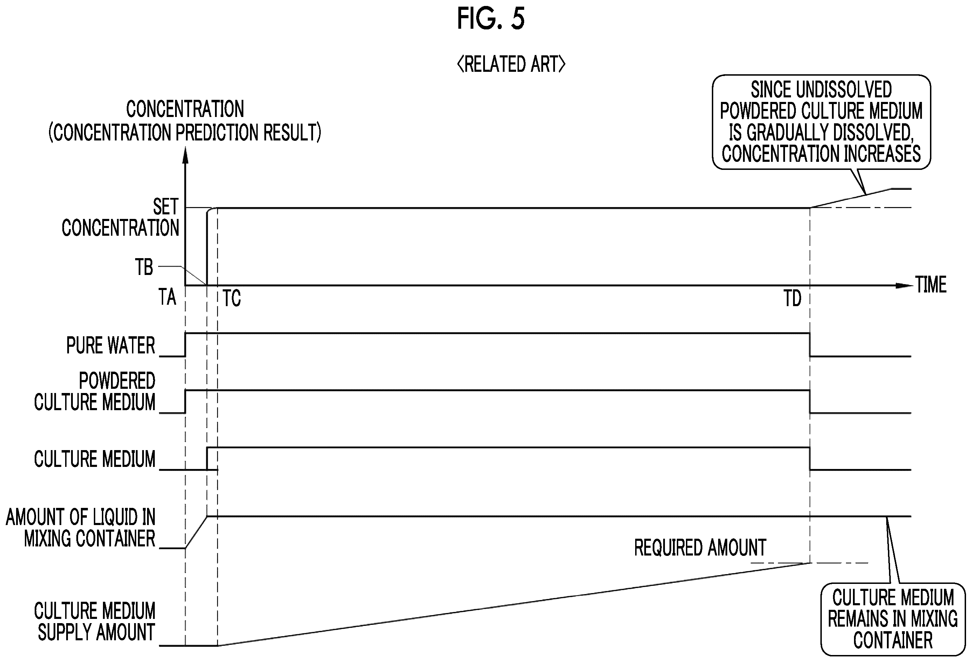

FIG. 5 shows processing in the related art. At a time point TA, in a case where the operator gives a preparation start instruction for the culture medium CM through the operation unit 20, the pure water supply pump 22 and the powdered culture medium supply machine 17 are driven under the control of the pure water supply pump drive control unit 65 and the powdered culture medium supply machine drive control unit 66, and the supply of the pure water PW and the powdered culture medium PM to the mixing container 16 is started.

At a time point TB, in a case where the amount of the liquid in the mixing container 16 reaches the set amount, although not shown, the stirrer 32 is driven under the control of the stirrer drive control unit 67. In addition, the culture medium supply pump 36 is driven under the control of the culture medium supply pump drive control unit 68. In this case, since the concentration of the culture medium CM in the mixing container 16 is not yet the set concentration, the flow path of the three-way valve 46 is set to the waste liquid line 47 side by the three-way valve drive control unit 70, and the waste liquid WL, which is the culture medium CM that is not at the set concentration, is discharged to the waste liquid recovery tank 18.

At a time point TC, in a case where the concentration of the culture medium CM in the mixing container 16 reaches the set concentration, the flow path of the three-way valve 46 is set to the culture tank 11 side by the three-way valve drive control unit 70, and the introduction of the culture medium CM having the set concentration into the culture tank 11 is started. The supply of the pure water PW and the powdered culture medium PM to the mixing container 16 and the supply of the culture medium CM from the mixing container 16 to the culture tank 11 are continued until the culture in the culture tank 11 is ended and the required amount of the culture medium CM is completely supplied from the mixing container 16 to the culture tank 11. The drive of the culture medium supply pump 36 may be started at the time point TC at which the concentration of the culture medium CM in the mixing container 16 reaches the set concentration, instead of the time point TB at which the amount of the liquid in the mixing container 16 reaches the set amount.

At a time point TD, in a case where the required amount of the culture medium CM is completely supplied from the mixing container 16 to the culture tank 11, the supply of the pure water PW and the powdered culture medium PM to the mixing container 16 and the supply of the culture medium CM from the mixing container 16 to the culture tank 11 are stopped. At this time point TD, the culture medium CM and the undissolved powdered culture medium PM remain in the mixing container 16. In the related art, the culture medium CM and the like remaining in the mixing container 16 are completely discarded without being supplied to the culture tank 11. Therefore, the powdered culture medium PM is wasted.

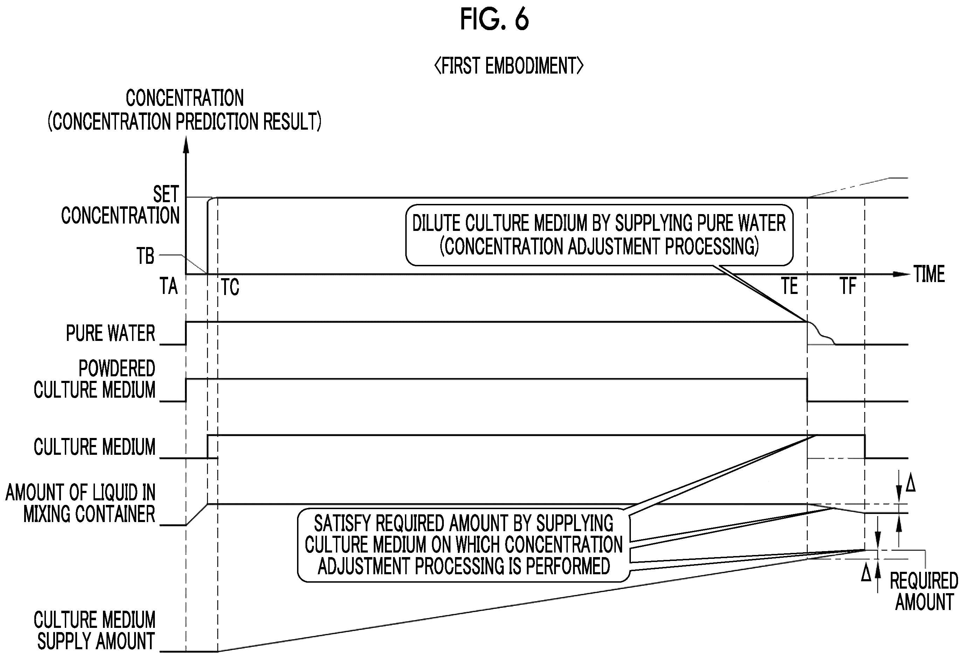

On the other hand, in the technology of the present disclosure, the culture medium CM remaining in the mixing container 16 is supplied to the culture tank 11 to add the required amount, thereby reducing the powdered culture medium PM that is wasted. However, since the undissolved powdered culture medium PM is gradually dissolved, the concentration of the culture medium CM remaining in the mixing container 16 is higher than the set concentration with the elapse of time, and thus the culture medium CM cannot be supplied to the culture tank 11 as it is. Therefore, in the technology of the present disclosure, for example, the concentration of the culture medium CM remaining in the mixing container 16 is maintained at the set concentration by performing the concentration adjustment processing shown in FIG. 6.

As shown in FIG. 6, at a time point TE, in a case where the integrated value of the outflow amount of the culture medium CM to the mixing container 16 is (the required amount - Δ), the supply of the powdered culture medium PM to the mixing container 16 is stopped. However, only the supply of the powdered culture medium PM is stopped, and the supply of the pure water PW to the mixing container 16 is still continued. That is, in the present first embodiment, as the concentration adjustment processing, processing of diluting the culture medium CM by supplying the pure water PW to the mixing container 16 is performed. In addition, not only the supply of the pure water PW to the mixing container 16 but also the supply of the set amount of the culture medium CM from the mixing container 16 to the culture tank 11 are still continued. The excess amount Δ is a value set in advance, is smaller than the set amount of the amount of the liquid in the mixing container 16, and is, for example, a value of 1/4 or more of the set amount of the amount of the liquid in the mixing container 16. In this case, the time point TE after the continuous supply of the powdered culture medium PM to the mixing container 16 is stopped is an example of a "case where the continuous supply of the powder to the mixing container is stopped" according to the technology of the present disclosure.

The pure water supply pump drive control unit 65 adjusts the supply amount of the pure water PW to the mixing container 16 by the pure water supply pump 22 such that the concentration prediction result 75 from the concentration prediction unit 69 is the set concentration. That is, the pure water supply pump drive control unit 65 performs the concentration adjustment processing according to the concentration prediction result 75. As a result, the increase in the concentration of the culture medium CM remaining in the mixing container 16 due to the undissolved powdered culture medium PM is suppressed, and the concentration of the culture medium CM remaining in the mixing container 16 is maintained at the set concentration. The culture medium CM remaining in the mixing container 16, which is maintained at the set concentration, is supplied from the mixing container 16 to the culture tank 11 by the culture medium supply pump 36 by the amount of the excess amount Δ, which is the required amount, under the control of the culture medium supply pump drive control unit 68. Then, in a case where the required amount of the culture medium CM is completely supplied from the mixing container 16 to the culture tank 11 at the time point TF, the supply of the culture medium CM from the mixing container 16 to the culture tank 11 is stopped.

As described above, the culture medium preparation device 10 comprises the CPU 51. As shown in FIG. 6, the pure water supply pump drive control unit 65 of the CPU 51 performs the concentration adjustment processing for maintaining the set concentration on the culture medium CM remaining in the mixing container 16 after the time point TE at which the continuous supply of the powdered culture medium PM to the mixing container 16 is stopped. The culture medium supply pump drive control unit 68 supplies the culture medium CM having the set concentration, on which the concentration adjustment processing is performed, to the culture tank 11 to satisfy the required amount of the culture medium CM having the set concentration. Therefore, it is possible to reduce the powdered culture medium PM that is wasted as compared with the related art in which the culture medium CM remaining in the mixing container 16 is completely discarded without being supplied to the culture tank 11. Therefore, it is possible to reduce the cost as compared with the related art.

As shown in FIG. 6, the pure water supply pump drive control unit 65 performs processing of diluting the culture medium CM by supplying the pure water PW to the mixing container 16 as the concentration adjustment processing. Therefore, the powdered culture medium PM that is not dissolved in the culture medium CM at the time point TE can also be effectively used, and the powdered culture medium PM that is wasted can be further reduced.

As shown in FIG. 6, the pure water supply pump drive control unit 65 performs the concentration adjustment processing according to the concentration prediction result 75. Therefore, the concentration of the culture medium CM remaining in the mixing container 16 can be more reliably maintained at the set concentration.

As shown in FIG. 4, the concentration prediction result 75 is based on the Raman spectral data 80 of the culture medium CM. The Raman spectral data 80 easily reflects the physical properties of the culture medium CM. Therefore, by using the Raman spectral data 80, the concentration prediction result 75 having high reliability can be obtained. The spectroscopic spectral data is not limited to the Raman spectral data 80 shown as an example. The physical property data may be infrared absorption spectral data, near infrared absorption spectral data, nuclear magnetic resonance spectral data, ultraviolet visible absorption spectroscopy (UV-Vis) spectral data, or fluorescence spectral data.

As shown in FIG. 1, the sensor unit 42 of the Raman spectrometer 41, which is a measuring instrument for the Raman spectral data 80, is installed in the mixing container 16. Therefore, the concentration of the culture medium CM in the mixing container 16 can be more accurately predicted. The flow cell may be connected to an appropriate location of the culture medium supply line 35, such as the upstream side of the filter 38, the sensor unit 42 may be attached to the flow cell, and the Raman spectral data 80 of the culture medium CM flowing through the flow cell may be measured.

As shown in FIG. 1, the solvent is the pure water PW, the powder is the powdered culture medium PM, and the solution is the culture medium CM. Therefore, the culture medium CM having a set concentration, which is indispensable for the culture, can be prepared at a lower cost than in the related art.

### [Second embodiment]

In the first embodiment, the process of diluting the culture medium CM by supplying the pure water PW to the mixing container 16 has been described as an example of the concentration adjustment processing, but the present disclosure is not limited thereto.

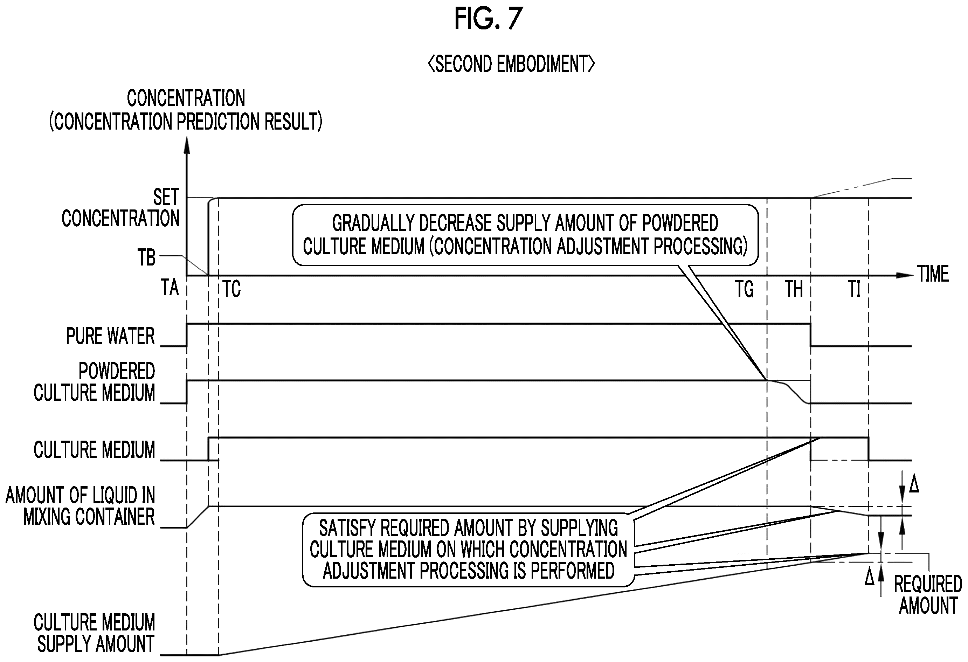

As shown in FIG. 7 as an example, in the second embodiment, at a time point TG before a time point TH at which the integrated value of the outflow amount of the culture medium CM to the mixing container 16 reaches (the required amount - Δ), the processing of gradually decreasing the supply amount of the powdered culture medium PM to the mixing container 16 by the powdered culture medium supply machine 17 under the control of the powdered culture medium supply machine drive control unit 66 is started as the concentration adjustment processing. The concentration adjustment processing of gradually decreasing the supply amount of the powdered culture medium PM is ended by the time point TH at which the integrated value of the outflow amount of the culture medium CM to the mixing container 16 reaches (the required amount - Δ). In this case, a time point immediately before the time point TH at which the continuous supply of the powdered culture medium PM to the mixing container 16 is stopped (between the time point TG and the time point TH) is an example of a "case where the continuous supply of the powder to the mixing container is stopped" according to the technology of the present disclosure.

Even during the concentration adjustment processing of gradually decreasing the supply amount of the powdered culture medium PM, the supply of the set amount of the pure water PW to the mixing container 16 is still continued. In addition, not only the supply of the set amount of the pure water PW to the mixing container 16 but also the supply of the set amount of the culture medium CM from the mixing container 16 to the culture tank 11 are still continued. The time points TG and TH can be calculated backward from a time point TI at which the required amount of the culture medium CM is completely supplied from the mixing container 16 to the culture tank 11.

In the second embodiment, the concentration prediction unit 69 outputs a concentration prediction result 75 to the powdered culture medium supply machine drive control unit 66. The powdered culture medium supply machine drive control unit 66 adjusts the supply amount of the powdered culture medium PM to the mixing container 16 by the powdered culture medium supply machine 17 such that the concentration prediction result 75 from the concentration prediction unit 69 reaches the set concentration. That is, the powdered culture medium supply machine drive control unit 66 performs the concentration adjustment processing according to the concentration prediction result 75. As a result, as in the first embodiment, the increase in the concentration of the culture medium CM remaining in the mixing container 16 due to the undissolved powdered culture medium PM is suppressed, and the concentration of the culture medium CM remaining in the mixing container 16 is maintained at the set concentration. The culture medium CM remaining in the mixing container 16, which is maintained at the set concentration, is supplied from the mixing container 16 to the culture tank 11 by the culture medium supply pump 36 by the amount of the excess amount Δ, which is the required amount, under the control of the culture medium supply pump drive control unit 68. Then, in a case where the required amount of the culture medium CM is completely supplied from the mixing container 16 to the culture tank 11 at the time point TI, the supply of the culture medium CM from the mixing container 16 to the culture tank 11 is stopped.

As described above, in the second embodiment, the powdered culture medium supply machine drive control unit 66 performs processing of gradually decreasing the supply amount of the powdered culture medium PM as the concentration adjustment processing. Even with the concentration adjustment processing, since the increase in the concentration of the culture medium CM due to the undissolved powdered culture medium PM is suppressed, as in the first embodiment, the amount of the powdered culture medium PM that is wasted can be reduced, and the cost can be reduced.

A time at which the culture in the culture tank 11 ends is reserved and set, and the time point TE of the first embodiment or the time points TG and TH of the second embodiment are calculated backward from the reserved and set time. Then, the concentration adjustment processing may be started at the calculated time point TE or TG.

The concentration adjustment processing may be performed with the supply amount of the pure water PW or the powdered culture medium PM set in advance without referring to the concentration prediction result 75. In this case, the supply amount of the pure water PW or the powdered culture medium PM is set based on various parameters such as the dissolution rate of the powdered culture medium PM and the amount of the undissolved powdered culture medium PM in a case where the concentration adjustment processing is started. In addition, in this case, the supply amount of the pure water PW or the powdered culture medium PM may be changed linearly or may be changed in a stepwise manner.

The method of measuring the concentration of the culture medium CM is not limited to the method using the spectroscopic spectral data such as the Raman spectral data 80 described as an example. The conductivity meter may be connected to the mixing container 16 or the culture medium supply line 35, and the conductivity measured by the conductivity meter may be converted into the concentration. Examples of the connection location of the conductivity meter include a location of the culture medium supply line 35 between the culture medium flow meter 37 and the hydrogen-ion exponent meter 45. Alternatively, the hydrogen-ion exponent measured by the hydrogen-ion exponent meter 45 may be converted into the concentration.

The method of measuring the timing at which the supply of the waste liquid of the culture medium CM is stopped and the supply to the culture tank 11 is started is not limited to the method based on the measurement result of the concentration. The time taken for the concentration of the culture medium CM to reach the set concentration is measured and stored in advance. Then, in a case where the elapsed time from the start of the continuous supply of the pure water PW and the powdered culture medium PM to the mixing container 16 is timed and the elapsed time reaches the stored time, the discard of the culture medium CM may be stopped and the supply of the culture medium CM to the culture tank 11 may be started.

In each of the embodiments, pure water PW is exemplified as the solvent and the purified water, but the present disclosure is not limited thereto. Distilled water or the like may be used. In addition, in each of the above-described embodiments, the culture medium CM is described as an example of the solution, but the present disclosure is not limited thereto. The solution may be a buffer solution. Therefore, the powder is not limited to the powdered culture medium PM described as an example, and may be a powder constituting the solid component of the buffer solution.

In each of the above-described embodiments, for example, as the hardware structure of the processing unit that executes various types of processing, such as the pure water supply pump drive control unit 65, the powdered culture medium supply machine drive control unit 66, the stirrer drive control unit 67, the culture medium supply pump drive control unit 68, the concentration prediction unit 69, and the three-way valve drive control unit 70, various processors described later can be used. As described above, the various processors include, in addition to the CPU 51 that is a general-purpose processor executing software (operation program 60) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute specific processing, such as an application specific integrated circuit (ASIC).

One processing unit may be configured by one of the various processors, or may be configured by a combination of two or more processors of the same type or different types (for example, a combination of a plurality of FPGAs and/or a combination of a CPU and an FPGA). In addition, a plurality of processing units may be configured by one processor.

As an example in which the plurality of processing units are configured by one processor, first, as represented by a computer such as a client and a server, there is a form in which one processor is configured by a combination of one or more CPUs and software and the processor functions as the plurality of processing units. Second, as represented by a system on chip (SoC) or the like, there is a form in which a processor that realizes the functions of the entire system including a plurality of processing units by one integrated circuit (IC) chip is used. In this manner, various processing units are configured by using one or more of the various processors as a hardware structure.

Further, as a hardware structure of the various processors, more specifically, an electrical circuit (circuitry) in which circuit elements such as semiconductor elements are combined can be used.

The technology according to the following supplementary notes can be understood from the above description.

### [Supplementary Note 1]

A solution preparation device that continuously supplies a solvent and a powder to a mixing container to continuously prepare a solution having a set concentration in the mixing container and that continuously supplies the solution having the set concentration from the mixing container to a processing container, the solution preparation device comprising:
a processor,
in which the processor is configured to:
   perform concentration adjustment processing for maintaining the set concentration on a solution remaining in the mixing container in a case where the continuous supply of the powder to the mixing container is stopped; and
   supply the solution having the set concentration, on which the concentration adjustment processing is performed, to the processing container to satisfy a required amount of the solution having the set concentration.

### [Supplementary Note 2]

The solution preparation device according to Supplementary Note 1,
in which the processor is configured to perform, as the concentration adjustment processing, processing of diluting the solution by supplying the solvent to the mixing container.

### [Supplementary Note 3]

The solution preparation device according to Supplementary Note 1,
in which the processor is configured to perform, as the concentration adjustment processing, processing of gradually decreasing a supply amount of the powder.

### [Supplementary Note 4]

The solution preparation device according to any one of Supplementary Notes 1 to 3,
in which the processor is configured to perform the concentration adjustment processing according to a measurement result of a concentration of the solution.

### [Supplementary Note 5]

The solution preparation device according to Supplementary Note 4,
in which the measurement result is based on spectroscopic spectral data of the solution.

### [Supplementary Note 6]

The solution preparation device according to Supplementary Note 5,
in which a measuring instrument for the spectroscopic spectral data is installed in the mixing container.

### [Supplementary Note 7]

The solution preparation device according to any one of Supplementary Notes 1 to 6,
in which the solvent is purified water, the powder is a powdered culture medium, and
the solution is a culture medium.

The technology of the present disclosure can also appropriately combine various embodiments and/or various modification examples described above. It goes without saying that the present disclosure is not limited to each of the embodiments described above, and various configurations can be employed without departing from the gist. Furthermore, the technology of the present disclosure extends to a storage medium that non-transitorily stores the program, and a computer program product including the program, in addition to the program.

The description content and the shown content described above are detailed descriptions of parts related to the technology of the present disclosure and are merely an example of the technology of the present disclosure. For example, the above description of the configuration, the function, the action, and the effect are the description of examples of the configuration, the function, the action, and the effect of the parts according to the technology of the present disclosure. Accordingly, it goes without saying that unnecessary parts may be deleted, new elements may be added, or replacements may be made with respect to the description content and the shown content described above within a range that does not depart from the gist of the technology of the present disclosure. Further, in order to avoid complication and facilitate understanding of the parts according to the technology of the present disclosure, the description related to common general knowledge not requiring special description in order to implement the technology of the present disclosure is omitted in the description content and the shown content described above.

In the present specification, "A and/or B" is synonymous with "at least one of A or B". That is, "A and/or B" means that it may be only A, only B, or a combination of A and B. In addition, in the present specification, in a case where three or more matters are expressed by being connected by "and/or", the same concept as "A and/or B" is applied.

All documents, patent applications, and technical standards described in the present specification are incorporated in the present specification by reference to the same extent as in a case where each of the documents, the patent applications, and the technical standards are specifically and individually indicated to be incorporated by reference.

## Claims

1. A solution preparation device that continuously supplies a solvent and a powder to a mixing container to continuously prepare a solution having a set concentration in the mixing container and that continuously supplies the solution having the set concentration from the mixing container to a processing container, the solution preparation device comprising:
a processor, the processor being configured to:
perform concentration adjustment processing for maintaining the set concentration on a solution remaining in the mixing container in a case where the continuous supply of the powder to the mixing container is stopped; and
supply the solution having the set concentration, on which the concentration adjustment processing is performed, to the processing container to satisfy a required amount of the solution having the set concentration.

2. The solution preparation device according to claim 1, wherein the processor is configured to perform, as the concentration adjustment processing, processing of diluting the solution by supplying the solvent to the mixing container.

3. The solution preparation device according to claim 1, wherein the processor is configured to perform, as the concentration adjustment processing, processing of gradually decreasing a supply amount of the powder.

4. The solution preparation device according to claim 1, wherein the processor is configured to perform the concentration adjustment processing according to a measurement result of a concentration of the solution.

5. The solution preparation device according to claim 4, wherein the measurement result is based on spectroscopic spectral data of the solution.

6. The solution preparation device according to claim 5, wherein a measuring instrument for the spectroscopic spectral data is installed in the mixing container.

7. The solution preparation device according to claim 1, wherein:
the solvent is purified water,
the powder is a powdered culture medium, and
the solution is a culture medium.

8. An operation method of a solution preparation device that continuously supplies a solvent and a powder to a mixing container to continuously prepare a solution having a set concentration in the mixing container and that continuously supplies the solution having the set concentration from the mixing container to a processing container, the method comprising:
performing concentration adjustment processing for maintaining the set concentration on a solution remaining in the mixing container in a case where the continuous supply of the powder to the mixing container is stopped; and
supplying the solution having the set concentration, on which the concentration adjustment processing is performed, to the processing container to satisfy a required amount of the solution having the set concentration.

9. An operation program of a solution preparation device that continuously supplies a solvent and a powder to a mixing container to continuously prepare a solution having a set concentration in the mixing container and that continuously supplies the solution having the set concentration from the mixing container to a processing container, the program causing a computer to execute a process comprising:
performing concentration adjustment processing for maintaining the set concentration on a solution remaining in the mixing container in a case where the continuous supply of the powder to the mixing container is stopped; and
supplying the solution having the set concentration, on which the concentration adjustment processing is performed, to the processing container to satisfy a required amount of the solution having the set concentration.
